# EUROPÄISCHE PATENTSCHRIFT

(11) **EP 0 705 268 B1**
(45) Veröffentlichungstag und Bekanntmachung des Hinweises auf die Patenterteilung: **22.01.1997**
(21) Anmeldenummer: 94921615.4
(22) Anmeldetag: 20.06.1994
(51) Int. Cl.: C07F 9/6571, C07C 45/50, C07F 9/6574, C07F 15/00

(54) **PHOSPHORHALTIGE CALIXARENE**
PHOSPHOROUS-CONTAINING CALIXARENES
CALIXARENES PHOSPHORES

(30) Priorität: 25.06.1993 DE 4321194
(43) Veröffentlichungstag der Anmeldung: 10.04.1996
(73) Patentinhaber: BASF AKTIENGESELLSCHAFT, 67056 Ludwigshafen (DE)
(72) Erfinder: PACIELLO, Rocco, D-67098 Bad Dürkheim (DE); RÖPER, Michael, D-67157 Wachenheim (DE); KNEUPER, Heinz-Josef, D-68163 Mannheim (DE); LANGGUTH, Ernst, D-67281 Kirchheim (DE); LORZ, Peter, Michael, D-68161 Mannheim (DE)
(86) Internationale Anmeldenummer: EP9401999
(87) Internationale Veröffentlichungsnummer: WO9500525

(56) Entgegenhaltungen:
- EP-A- 0 353 770
- EP-A- 0 487 036
- US-A- 4 416 829
- PHOSPHORUS, SULFUR SILICON RELAT. ELEM. (PSSLEC,10426507);93; VOL.75 (1-4); PP.253-6, SOUTH. METHODIST UNIV.;DEP. CHEM.; DALLAS; 75275; TX; USA (US) Khasnis D V et al 'Chemistry in molecular baskets: variable coordination of phosphorus in calix[4]arenes'

## Beschreibung

Die vorliegende Erfindung betrifft phosphorhaltige Calixarene.

Unter Carbonylierungsreaktionen versteht man die Produktion von sauerstoffhaltigen Produkten durch Umsetzung einer organischen Verbindung mit Kohlenmonoxid und vorzugsweise einem weiteren Reaktionspartner - vor allem Wasserstoff - in Anwesenheit eines Katalysators. Eine technisch besonders wichtige Reaktion ist die Hydroformylierung von Olefinen durch Umsetzung mit Kohlenmonoxid und Wasserstoff unter Bildung von Aldehyden, die ein Kohlenstoffatom mehr als die Edukte enthalten. Als Katalysatoren verwendet man Gruppe-VIII-Übergangsmetallkomplexe, die phosphorhaltige Liganden, z. B. Phosphite enthalten (s. J. Falbe, New Synthesis with Carbon Monoxide, Springer Verlag, New York 1980).

Neben Cobaltkatalysatoren haben in den letzten Jahren zunehmend Rhodiumkatalysatoren zur Hydroformylierung niederer α-Olefine Bedeutung erlangt, da sie eine Reaktionsführung bei einem niedrigeren Druck zulassen. Als Phosphorligand wird in der Regel Triphenylphosphin im Überschuß verwendet, wobei ein hohes Ligand/ Rhodium-Verhältnis erforderlich ist, um die Selektivität der Reaktion zum kommerziell erwünschten n-Aldehydprodukt zu erhöhen.

In den letzten Jahren wurde der Versuch unternommen, wirksamere Phosphorliganden für die Hydroformylierung zu finden. Neben unterschiedlich substituierten Phosphiten wurden auch Phosphite auf ihre Eignung als Katalysatoren untersucht. Phosphite ergeben bei ihrer Koordination an ein Übergangsmetallzentrum Katalysatoren von gesteigerter Aktivität, doch ist das Standzeitverhalten dieser Katalysatorsysteme wegen der starken Hydrolyseempfindlichkeit der Phosphitliganden unbefriedigend. Eine drastisch verringerte Hydrolyseempfindlichkeit sollen durch Bisaryldiole substituierte chelatisierende Polyphosphite aufweisen, wie sie in EP-A 21 46 22 beschrieben werden. Die Rhodiumkomplexe dieser Liganden sollen äußerst aktive Hydroformylierungskatalysatoren bilden. EP-A 213 639 beschreibt Chelat-Bisphosphite, die an einem Phosphoratom Diorgano- in der zweiten Phosphoratom Triorganophosphit-Funktionalität aufweisen. Aus EP-A 155 508 ist ferner die Verwendung von bisaryldiolsubstituierten Monophosphiten bei der rhodiumkatalysierten Hydroformylierung von sterisch gehinderten Olefinen, z. B. Isobuten bekannt. In EP-A 472 071 werden Chelat-Bisphosphit-Liganden offenbart, in denen Bisaryldiole über Bisphosphitester-Brücken mit Alkandiolen oder o-Aryldiolen verknüpft sind. Diol- und Triol-substituierte Monophosphite und ihr Einsatz bei der Hydroformylierung werden schließlich in EP-A 149 894, EP-A 96 988 und EP-A 96 986, beschrieben.

Bei der Hydroformylierung von Olefinen bilden sich, je nach dem an welcher Position der Doppelbindung das Kohlenmonoxid addiert wird, geradkettige Aldehyde, die als n-Aldehyde bezeichnet werden, oder verzweigtkettige Aldehyde, sogenannte iso-Aldehyde. Dies ist schematisch in Gleichung (1) dargestellt:

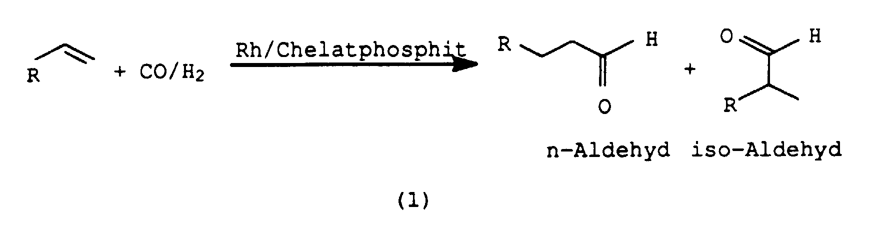

Es ist nun aber in der Regel erwünscht, daß der Anteil des n-Al-dehyds den des iso-Aldehyds im Hydroformylierungsprodukt, im folgenden als n-Anteil bezeichnet, möglichst groß ist, da bei der Weiterverarbeitung der Aldehyde zu Weichmacher-Alkoholen und Weichmachern, die n-Aldehyde zu Produkten mit besonders günstigen Weichmachereigenschaften führen (vgl. z. B. US-A 4 426 542).

Bei Anwendung der Chelat-Bisphosphit-Liganden des zuvor genannten Standes der Technik können bei der Rhodium-katalysierten Hydroformylierung von Olefinen Hydroformylierungsprodukte mit einem sehr hohen n-Anteil von bis zu 96 % erhalten werden.

Der vorliegenden Verbindung lag nun die Aufgabe zugrunde, Liganden für die Rhodium- oder Ruthenium-katalysierte Hydroformylierung von Olefinen zu finden, mit deren Hilfe sich der n-Anteil im Hydroformylierungsprodukt noch weiter erhöhen läßt.

Dementsprechend wurden phosphorhaltige Calixarene der allgemeinen Formel I 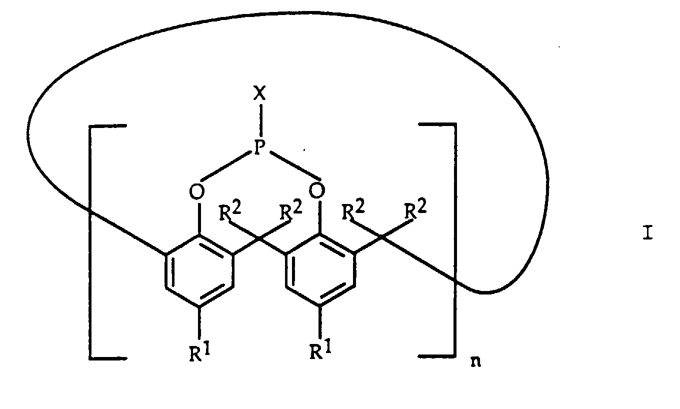 in der n eine ganze Zahl von 2 bis 4 ist,
- R¹: Wasserstoff, eine C₁- bis C₂₀-Alkylgruppe, eine C₁- bis C₂₀-Alkoxygruppe, eine Sulfonatgruppe oder eine Carboxylatgruppe bedeutet, die Reste
- R²: gleich oder verschieden sind und für Wasserstoff oder eine C₁- bis C₂₀-Alkylgruppe stehen, und
- X: Wasserstoff, eine C₁- bis C₂₀-Alkylgruppe, eine C₁- bis C₂₀-Alkoxygruppe oder eine unsubstituierte oder mit 1 bis 3 C₁- bis C₂₀-Alkylgruppen, C₁- bis C₂₀-Alkoxygruppen, Sulfonatgruppen, Carboxylatgruppen, C₁- bis C₂₀-Alkyl-thiogruppen und/oder C₂- bis C₂₀-Dialkylaminogruppen substituierte Phenyl- oder Phenoxygruppe ist.

Des weiteren wurde ein Verfahren zur Herstellung der phosphorhaltigen Calixarene der Formel I gefunden, das dadurch gekennzeichnet ist, daß man ein Calixaren der allgemeinen Formel IV 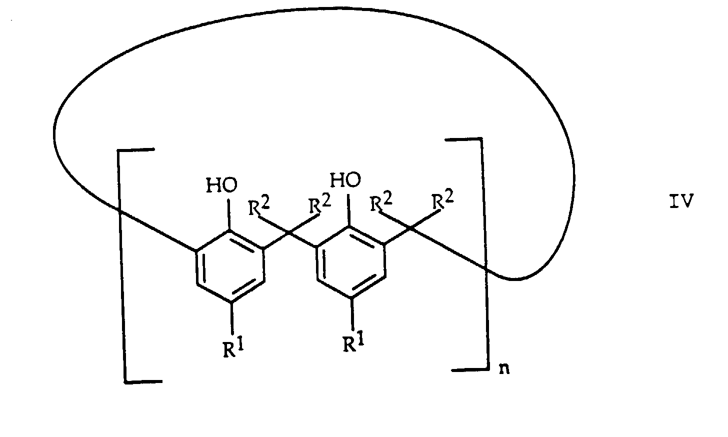 in der n und die Reste R¹ und R² die obengenannte Bedeutung haben, mit einer Phosphorverbindung der allgemeinen Formel V

XPHal₂ V

in der X die obengenannte Bedeutung hat und Hal für Fluor, Chlor, Brom oder Jod steht, in Gegenwart einer Base umsetzt.

Weiterhin wurde ein Verfahren zur Herstellung von Aldehyden durch die Hydroformylierung von C₂- bis C₂₀-Olefinen mittels eines CO/H2-Gasgemisches in Gegenwart von Rhodium- oder Ruthenium-Carbonylkomplexen mit einem phosphorhaltigen Liganden und in Gegenwart eines Lösungsmittels gefunden, das dadurch gekennzeichnet ist, daß man als Liganden die phosphorhaltigen Calixarene der Formel I verwendet.

Bei den erfindungsgemäßen phosphorhaltigen Calixarenen handelt es sich um cyclische Phosphite und Phosphonite von hydroxylierten Calixarenen.

In den erfindungsgemäßen phosphorhaltigen Calixarenen der allgemeinen Formel I kann der Rest R¹ Wasserstoff oder eine C₁- bis C₂₀-, vorzugsweise eine C₁- bis C₆-Alkylgruppe, eine C₁- bis C₂₀-, vorzugsweise eine C₁- bis C₆-Alkoxygruppe, eine Sulfonatgruppe oder eine Carboxylatgruppe sein. Besonders bevorzugt ist als Rest R¹ die tert.-Butylgruppe, die Sulfonatgruppe oder die Carboxylatgruppe.

Die Reste R² in den erfindungsgemäßen phosphorhaltigen Calixarenen der Formel I können gleich oder verschieden sein und Wasserstoff oder C₁- bis C₂₀-, vorzugsweise eine C₁- bis C₆-Alkylgruppe bedeuten. Besonders bevorzugt ist R² Wasserstoff.

Der Rest X in den erfindungsgemäßen phosphorhaltigen Calixarenen der Formel I kann Wasserstoff, eine C₁- bis C₂₀-, vorzugsweise eine C₁- bis C₆-Alkylgruppe, eine C₁- bis C₂₀-, vorzugsweise eine C₁- bis C₆-Alkoxygruppe oder eine unsubstituierte oder mit 1 bis 3, vorzugsweise mit 1 oder 2, C₁- bis C₂₀-, vorzugsweise C₁- bis C₆-Alkylgruppen, C₁- bis C₂₀-, vorzugsweise C₁- bis C₆-Alkoxygruppen, C₁- bis C₂₀-, vorzugsweise C₁- bis C₆-Alkylthiogruppen, C₂- bis C₂₀-, vorzugsweise C₂- bis C₁₀-Dialkylaminoqruppen, Sulfonatgruppen oder Carboxylatgruppen substituierte Phenyl- oder Phenoxygruppe sein. Bevorzugte phosphorhaltige Calixarene sind solche, in denen die Gruppe X eine Phenoxygruppe der allgemeinen Formel II ist 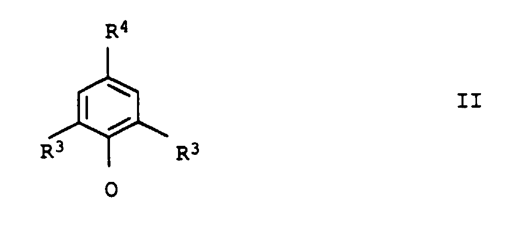 in der die Reste R³ und R⁴ gleich oder verschieden sein können, und für Wasserstoff, für C₁- bis C₂₀-, vorzugsweise für C₁- bis C₆-Alkylgruppen, für C₁- bis C₂₀-, vorzugsweise für C₁- bis C₆-Alkoxygruppen, für C₁- bis C₂₀-, vorzugsweise für C₁- bis C₆-Alkylthiogruppen und/oder für C₂- bis C₂₀-, vorzugsweise C₂-bis C₁₀-Dialkylaminogruppen stehen können, wobei der Rest R⁴ zusätzlich noch für eine Sulfonat- oder Carboxylatgruppe stehen kann.

Die Sulfonat- und Carboxylatgruppen in den erfindungsgemäßen Calixarenderivaten können sowohl in protonierter Form, d. h. als Sulfonsäure- oder Carbonsäuregruppen, vorliegen, bevorzugt sind solche Calixarene, in denen diese Gruppen in Salzform, beispielsweise als Alkalimetall-, Erdalkalimetall- oder Oniumsalz, insbesondere als Ammonium- oder Phosphoniumsalz vorliegen. Das Vorliegen von Sulfonat- oder Carboxylatgruppen in den erfindungsgemäßen phosphorhaltigen Calixarenen erhöht deren Wasserlöslichkeit und fördert auf diese Weise die Durchführung der Hydroformylierung in wäßrigem Medium.

Ein besonders bevorzugtes phosphorhaltiges Calixaren ist in Formel III dargestellt.

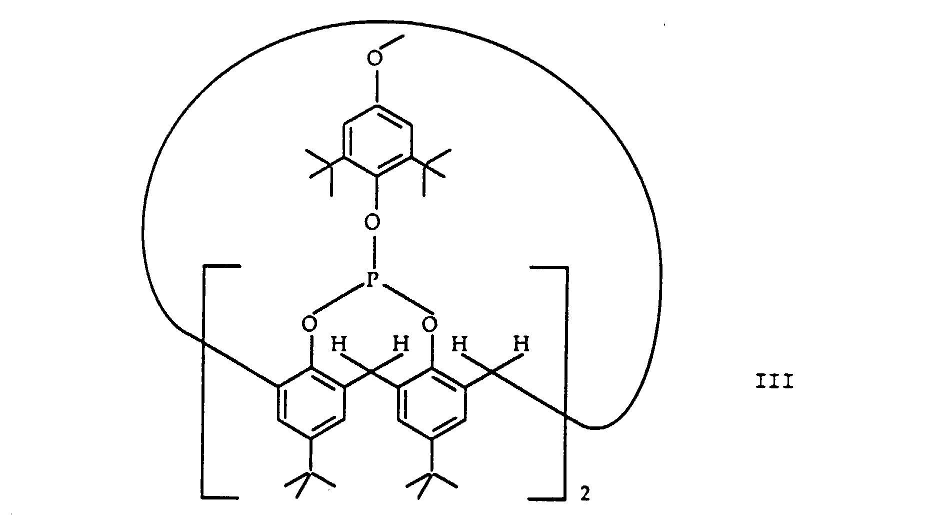

Die erfindungsgemäßen phosphorhaltigen Calixarene können durch die Umsetzung von Hydroxylgruppen-tragenden Calixarenen der allgemeinen Formel IV 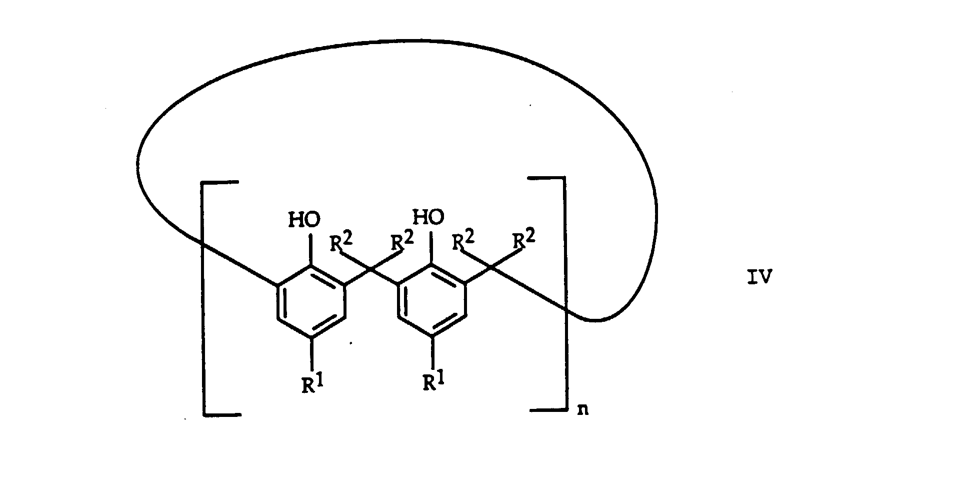 in der n und die Reste R¹ und R² die obengenannte Bedeutung haben, mit einer Phosphorverbindung der allgemeinen Formel V

XPHal₂ V

in der der Rest X die obengenannte Bedeutung hat und Hal für Fluor, Chlor, Brom oder Jod, vorzugsweise für Chlor, steht, in Gegenwart einer Base erhalten werden.

Als Calixarene werden cyclische Kondensationsprodukte aus parasubstituierten Phenolen und Aldehyden, vorzugsweise Formaldehyd, bezeichnet (vgl. Chemie in unserer Zeit 25, 195 (1991)). Sie können nach an sich bekannten Verfahren hergestellt werden, wie sie beispielsweise in Gutsche, "Calixarenes", Kapitel 2, S. 27 bis 66, The Royal Society of Chemistry, Cambridge 1989 beschrieben sind.

Die zur Herstellung der Calixarenphosphite benötigten phosphorhaltigen Verbindungen der Formel V können nach an sich bekannten Verfahren (vgl. Houben Weyl, Methoden der Org. Chemie Band XII/2, Kapitel 2, Thieme, Stuttgart 1964), beispielsweise durch die Umsetzung eines Phosphortrihalogenids, wie Phosphortrichlorid, Phosphortribromid oder Phosphortriodid mit einem Alkohol oder Phenol in Gegenwart einer Base, wie Alkalimetall oder Erdalkalimetallhydroxiden oder -Carbonaten oder vorzugsweise tert. Aminen, hergestellt werden.

Die zur Herstellung der Calixarenphosphonite benötigten phosphorhaltigen Verbindungen der Formel V können ebenfalls nach bekannten Methoden (vgl. Houben-Weyl, Methoden der Org. Chemie Band XII/1, S. 302-318, Thieme, Stuttgart 1963), beispielsweise durch die Umsetzung von eines Phosphortrihalogenids, wie Phosphortrichlorid, mit aromatischen Verbindungen, wie Benzol, in Gegenwart von Friedel-Crafts-Katalysatoren, wie Aluminiumchlorid, erzeugt werden.

Zur Herstellung der erfindungsgemäßen Calixarenderivate werden die betreffenden Calixarene IV mit vorzugsweise stöchiometrischen Mengen der phosphorhaltigen Verbindung V pro Monomereinheit des eingesetzten Calixarens umgesetzt. Als Monomereinheit des Calixarens wird die Formeleinheit, wie sie in der eckigen Klammer gemäß Formel IV dargestellt ist, definiert.

Die Umsetzung des Calixarens IV mit der Phosphorverbindung V wird in Gegenwart einer Base durchgeführt.

Als Basen können Mineralbasen, beispielsweise Alkalimetall- oder Erdalkalimetalloxide, Alkalimetall- oder Erdalkalimetallhydroxide oder Alkalimetall- oder Erdalkalimetallcarbonate, verwendet werden, bevorzugt wird die Umsetzung jedoch in Gegenwart tertiärer Amine, vorzugsweise tertiärer aliphatischer Amine mit 3 bis 30 Kohlenstoffatomen, beispielsweise mit Trimethylamin, Triethylamin, Tri-n-propylamin, Ethyldiisopropylamin, Triisopropylamin, Tributylamin usw., durchgeführt. Die Base wird bezogen auf die Menge der eingesetzten Phosphorverbindung V in einem Molverhältnis von 1 bis 200, vorzugsweise von 1,5 bis 100 und besonders bevorzugt von 2 bis 10 zugesetzt.

Die Herstellung der erfindungsgemäßen Calixarenderivate erfolgt zweckmäßigerweise in einem Lösungsmittel. Als Lösungsmittel können alle Lösungsmittel verwendet werden, die sich unter den Reaktionsbedingungen inert verhalten, vorzugsweise aromatische Kohlenwasserstoffe, wie Benzol, Toluol, Xylol, oder Ether, wie Diethylether, Diisopropylether, Methyl-tert.-Butylether, Tetrahydrofuran, Dioxan, Ethylenglykoldimethylether usw.

Die Umsetzung wird im allgemeinen bei Temperaturen von -40°C bis 100°C durchgeführt. Der angewandte Druck ist für die Umsetzung im allgemeinen nicht kritisch, zweckmäßigerweise wird aber bei Atmosphärendruck oder unter dem Eigendruck des Reaktionssystems gearbeitet.

Die erfindungsgemäßen phosphorhaltigen Calixarene dienen als Liganden für Rhodium und Ruthenium bei der von Rhodium oder Ruthenium katalysierten Hydroformylierung von C₃- bis C₂₀-Olefinen. Als Olefine können Olefine mit interner Doppelbindung verwendet werden, bevorzugt werden nach dem vorliegenden Verfahren α-Olefine hydroformyliert.

Das Rhodium und das Ruthenium werden in an sich üblicher Weise in Form von Salzen, beispielsweise als Rhodium- oder Rutheniumacetat, oder -acetonylacetonat, als Rhodiumoxid oder Rutheniumoxid oder als Rhodium- oder Rutheniumcarbonyle in das erfindungsgemäße Verfahren eingebracht. Die Art der Verbindung in welcher das Rhodium oder Ruthenium im erfindungsgemäßen Verfahren eingesetzt wird, ist im allgemeinen nicht kritisch, da diese Verbindungen allesamt unter den angewandten Hydroformylierungsbedingungen in Gegenwart des CO/H₂-Reaktionsgases in die katalytisch aktive, homogen im Hydroformylierungsmedium gelöste Rhodium- oder Rutheniumspecies umgewandelt werden, die dann von den phosphorhaltigen Calixarenen komplexiert und stabilisiert werden. Die chemische Natur dieser katalytisch aktiven Rhodium- und Rutheniumspecies konnte nicht abschließend aufgeklärt werden, gleichwohl es in der Fachwelt eine Reihe von Vermutungen bezüglich ihrer chemischen Struktur gibt, die aber letztendlich alle noch nicht bewiesen werden konnten.

Da die katalytisch aktiven Rhodium- oder Rutheniumverbindungen echte Katalysatoren sind, d. h. bei der Umsetzung praktisch nicht verbraucht werden und als solche eine hohe Aktivität haben, reichen sehr geringe Mengen an Rhodium- oder Rutheniumverbindungen aus, um die Hydroformylierung mit zufriedenstellenden Umsätzen zu katalysieren. Im allgemeinen beträgt das Rhodium- bzw. Ruthenium-Molverhältnis bezüglich des eingesetzten Olefins im stationären Zustand der Umsetzung 1:1000 bis 1:50000 vorzugsweise 1:1000 bis 1:5000.

Die erfindungsgemäßen phosphorhaltigen Calixarene werden bezüglich des eingesetzten Rhodiums oder Rutheniums im Molverhältnis Calixaren/Rhodium bzw. Ruthenium von im allgemeinen 1 : 1 bis 100 : 1, vorzugsweise von 1:1 bis 20:1 und besonders bevorzugt von 1:1 bis 5:1 eingesetzt.

Das CO/H₂-Molverhältnis des der Hydroformylierung zugeführten CO/H₂-Gemisches kann 20:1 bis 1:20, vorzugsweise 1:1 bis 20:1 und besonders bevorzugt 1:1 betragen.

Das erfindungsgemäße Hydroformylierungsverfahren wird im allgemeinen in Gegenwart eines Lösungsmittels durchgeführt. Als Lösungsmittel können praktisch alle Lösungsmittel verwendet werden, die sich unter den Bedingungen der Hydroformylierung inert verhalten, z. B. Kohlenwasserstoffe, Ester oder Alkohole, bevorzugt werden jedoch die Aldehyde, die bei der Hydroformylierung des betreffenden Olefins entstehen, als Lösungsmittel bei der Hydroformylierung verwendet. Ein anderes besonders bevorzugtes Lösungsmittel sind sogenannte "Hochsieder", das sind Gemische aus hochsiedenden Verbindungen, wie sie während der Hydroformylierungsreaktion als Nebenprodukt in einer Reihe von Nebenreaktionen, wie Aldolkondensationen, Eliminierungen, Disproportionierungen und Hydrierungen, aus den bei der Hydroformylierung entstandenen Aldehyden gebildet werden. Eine Reihe solcher Hochsieder und die Art und Weise ihrer Entstehung werden in US-A 4 148 830 für den Fall der Herstellung von Butyraldehyd exemplarisch beschrieben.

Das erfindungsgemäße Verfahren wird im allgemeinen bei Temperaturen von 30 bis 150°C, vorzugsweise von 60 bis 130°C, besonders bevorzugt von 90 bis 110°C und bei einem Druck von im allgemeinen 1 x 10³ bis 1 x 10⁷ Pa, vorzugsweise von 1 x 10⁵ bis 5 x 10⁶ Pa und besonders bevorzugt von 5 x 10⁵ bis 3 x 10⁶ Pa ausgeführt.

Das erfindungsgemäße Hydroformylierungsverfahren kann diskontinuierlich ausgeübt werden, vorzugsweise wird es kontinuierlich betrieben. Dabei können an sich bekannte Verfahrenstechniken, wie sie beispielsweise in US-A 4 148 830, US-A 4 329 511 oder EP-A 313 559 beschrieben sind, angewandt werden.

Mit den erfindungsgemäßen phosphorhaltigen Calixarenen als Liganden für die katalytisch aktiven Rhodium- oder Rutheniumverbindungen gelingt es im erfindungsgemäßen Hydroformylierungsverfahren überraschenderweise den n-Anteil im Hydroformylierungsprodukt bis auf 99,5 % zu steigern.

### Beispiele

Herstellung des phosphorhaltigen Calixarens III 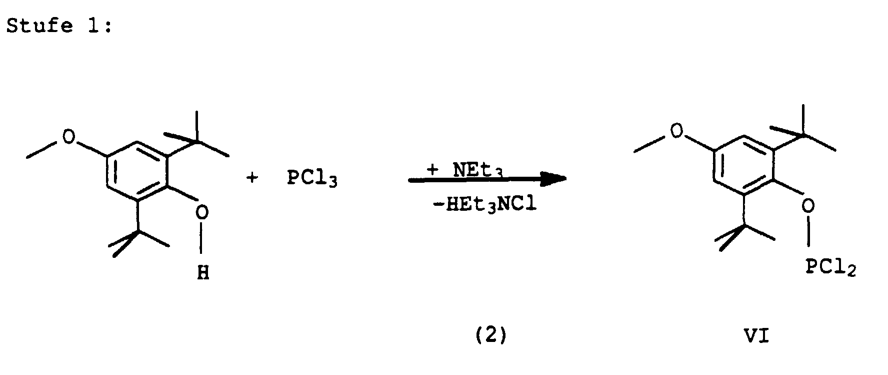 23,6 g (0,1 Mol) 3,5-Di-tert.-Butyl-4-Hydroxyanisol (im Handel erhältlich oder nach J. Am. Chem. Soc. 77, 1672 (1955) herstellbar) wurden in 500 ml Toluol gelöst und 50 ml Toluol aus dieser Mischung abdestilliert. Die abgekühlte Lösung wurde bei Raumtemperatur mit 68,2 ml (0,5 Mol) Triethylamin ersetzt und anschließend zu einer auf -40°C vorgekühlten Lösung von 8,8 ml (0,1 Mol) Phosphortrichlorid in 1 l Toluol dosiert. Die Lösung wurde langsam auf Raumtemperatur erwärmt, 1 Std. bei Raumtemperatur gerührt und anschließend noch 10 Stunden auf 100°C erhitzt. Die so erhaltene Lösung der Verbindung VI wurde in dieser Form der Stufe 2 zugeführt. 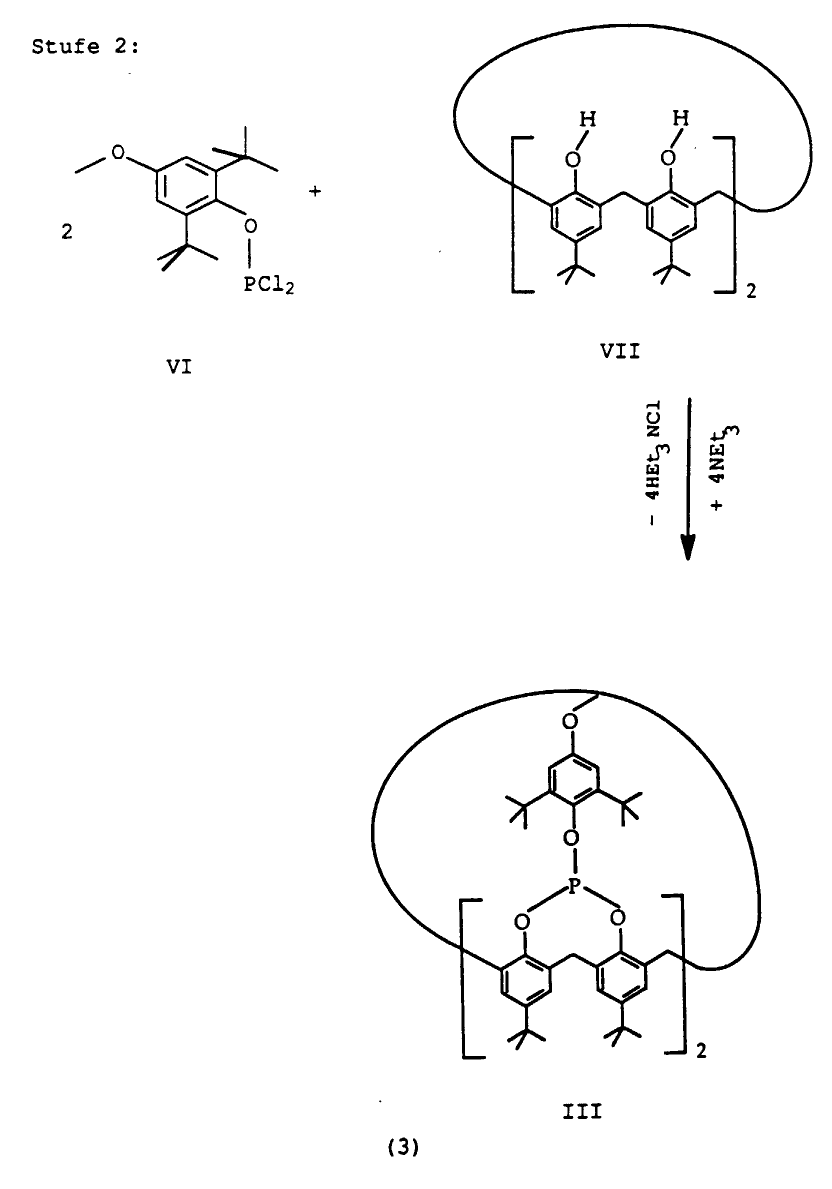 32,4 g (0,05 Mol) Calix-4-Aren VII (erhältlich nach: Gutsche "Calixarenes", Kapitel 2, Seite 27 - 66) wurden in Pulverform zur Lösung der Verbindung VI aus der 1. Stufe gegeben und dieses Gemisch auf -40°C abgekühlt. Zu dieser Mischung wurde eine Lösung von 136,5 ml (1 Mol) Triethylamin in 500 ml Toluol dosiert. Die Reaktionsmischung wurde langsam auf Raumtemperatur erwärmt, 1 Std. bei Raumtemperatur und weitere 10 Std. bei 100°C gerührt. Anschließend wurde das ausgefallene Triethylammoniumchlorid von der Reaktionslösung abfiltriert und mit Toluol gewaschen. Aus den vereinigten Toluolextrakten wurde das Toluol destillativ entfernt, wobei ein hellbrauner, zähflüssiger Rückstand zurückblieb. Dieser wurde mit Pentan gewaschen und anschließend mit 400 ml Acetonitril unter Rückfluß extrahiert. Nachdem sich ein Teil des Rückstandes gelöst hatte, wurde das übriggebliebene Pulver abfiltriert, mit Pentan gewaschen und getrocknet.

Die ¹H- und ¹³C-NMR-Spektren belegten die Struktur gemäß Formel III. Im ³¹P-NMR-Spektren wurde ein Signal bei 121 ppm, bezogen auf Phosphorsäure als Standard, beobachtet.

Elementaranalyse [Ergebnis (Theorie)]: C 75,5 % (76,2 %); H 8,3 % (8,9 %); 0 10,9 % (9,9 %); P 5,3 % (5,1 %).

Das Massenspektrum von III hatte als Hauptkomponente den Molekülpeak bei 1176 Dalton.

### Beispiel 2

### Hydroformylierung von 1-Octen

In einem 0,35 l Autoklaven wurde ein Gemisch aus 56,9 g 1-Octen (508 mmol), 0,032 g (0,124 mmol) Rhodium in Form der Komplexverbindung Rh(CO)₂(acac) (acac - Acetylacetonat) und 0,7098 g (0,604 mmol) der Verbindung III in 70 ml Texanol® (2,2,4-Trimethylpentan-1,3-diolmonoisobutyrat) auf 100°C erhitzt und durch Einleiten eines CO/H₂-Gemisches (Volumenverhältnis CO/H₂ = 1/1) in den Autoklaven ein Druck von 2 x 10⁶Pa eingestellt, der während der gesamten Umsetzungsdauer durch Nachpressen dieses Gasgemisches konstant gehalten wurde. Nach einer Reaktionszeit von 8 Stunden wurde das Hydroformylierungsgemisch gaschromatographisch analysiert. Das Ergebnis dieser Analyse ist in der Tabelle dargestellt.

**Tabelle**

| | | |
|---|---|---|
| int. C₈-Olefine | 3,41 Gew.-% | 0,0386 Mol |
| 1-Octen | 16,58 " | 0,1879 Mol |
| Oktan | 7,84 " | 0,0873 Mol |
| 2-Propylhexanal | 0,00 " | |
| 2-Ethylheptanal | 0,00 " | |
| 2-Methyloctanal | 0,11 " | 0,0010 Mol |
| n-Nonanal | 21,84 " | 0,1952 Mol |
| Texanol | 45,8 " | |
| Sonstige | 0,0 " | |
| Summe | 95,6 " | |
| | | |
| Umsatz | | 63 Mol.-% |
| Nonanal Ausbeute | | 39 Mol.-% |
| Oktan Ausbeute | | 17 Mol.-% |
| | | |
| Selektivität (berechnet auf umgesetztes 1-Octen) | | |
| | | |
| Nonanale | 61 % | |
| Oktan | 27 % | |
| interne Olefine | 12 % | |

Das Molverhältnis von 1-Nonanal zu den gesamten Nonanalen betrug 99,5 : 0,5.

Der Umsatz kann durch eine Erhöhung der Reaktionszeit weiter erhöht werden, ohne daß dabei die n-Aldehyd-Selektivität des Katalysators abnimmt.

## Patentansprüche

1. Phosphorhaltige Calixarene der allgemeinen Formel I 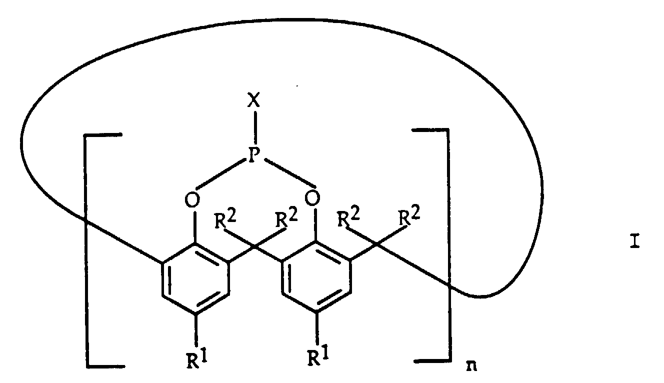 in der n eine ganze Zahl von 2 bis 4 ist,
R¹ Wasserstoff, eine C₁- bis C₂₀-Alkylgruppe, eine C₁- bis C₂₀-Alkoxygruppe, eine Sulfonatgruppe oder eine Carboxylatgruppe bedeutet, die Reste
R² gleich oder verschieden sind und für Wasserstoff oder eine C₁- bis C₂₀-Alkylgruppe stehen und
X Wasserstoff, eine C₁- bis C₂₀-Alkylgruppe, eine C₁- bis C₂₀-Alkoxygruppe oder eine unsubstituierte oder mit 1 bis 3 C₁- bis C₂₀-Alkylgruppen, C₁- bis C₂₀-Alkoxygruppen, Sulfonatgruppen, Carboxylatgruppen, C₁- bis C₂₀-Alkylthio- und/oder C₂- bis C₂₀-Dialkylaminogruppen substituierte Phenyl- oder Phenoxygruppe ist.

2. Phosphorhaltige Calixarene nach Anspruch 1, in denen der Rest X eine Phenyloxygruppe der allgemeinen Formel II 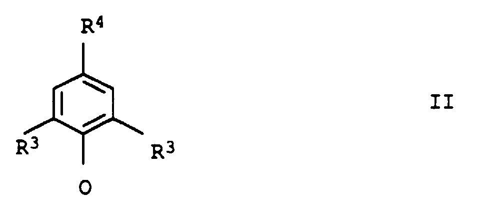 ist, in der die Reste R³ und der Rest R⁴ gleich oder verschieden sind und für Wasserstoff, C₁-C₂₀-Alkylgruppen, C₁- bis C₂₀-Alkoxygruppen, C₁- bis C₂₀-Alkylthiogruppen und/oder für C₂- bis C₂₀-Dialkylaminogruppen stehen und in der Rest R⁴ zusätzlich noch für eine Sulfonat- oder Carboxylatgruppe steht.

3. Phosphorhaltige Calixarene nach Anspruch 1, in denen der Rest R¹ Wasserstoff, eine C₁- bis C₆-Alkylgruppe, eine C₁- bis C₆-Alkoxygruppe, eine Sulfonatgruppe oder eine Carboxylatgruppe ist, der Rest R² für Wasserstoff steht und X die in Anspruch 2 genannte Bedeutung hat.

4. Phosphorhaltiges Calixaren nach Anspruch 1, der Formel III

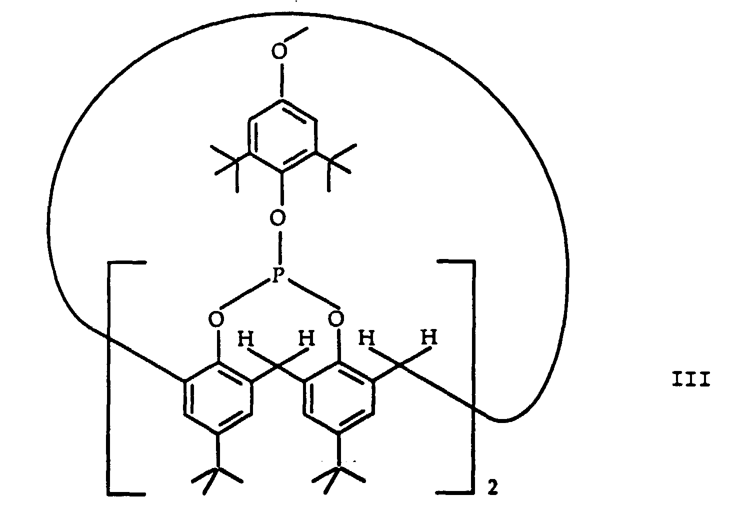

5. Verfahren zur Herstellung der phosphorhaltigen Calixarene gemäß Anspruch 1, dadurch gekennzeichnet, daß man ein Calixaren der allgemeinen Formel IV 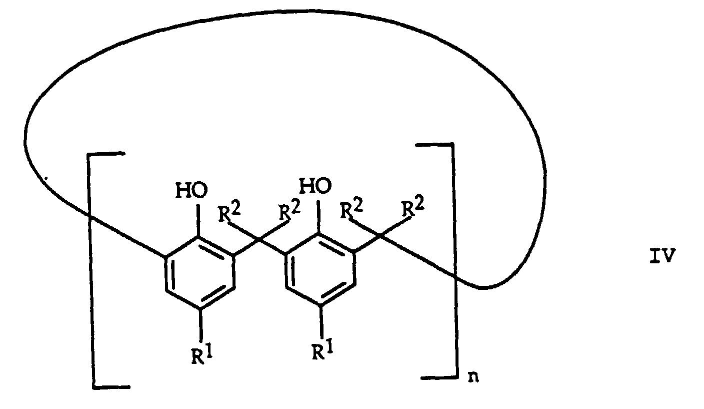 in der n und die Reste R¹ und R² die in Anspruch 1 genannte Bedeutung haben mit einer Phosphorverbindung der allgemeinen Formel V
XPHal₂ V
in der X die in Anspruch 1 genannte Bedeutung hat und Hal für Fluor, Chlor, Brom und Jod steht, in Gegenwart einer Base umsetzt.

6. Verfahren nach Anspruch 5, dadurch gekennzeichnet, daß man als Base ein C₃- bis C₃₀-Trialkylamin verwendet.

7. Verfahren zur Herstellung von Aldehyden durch die Hydroformylierung von C₃- bis C20-Olefinen mittels eines CO/H₂-Gasgemisches in Gegenwart von Rhodium- oder Ruthenium-Carbonylkomplexen mit einem phosphorhaltigen Liganden und in Gegenwart eines Lösungsmittels, dadurch gekennzeichnet, daß man als Liganden die phosphorhaltigen Calixarene gemäß den Ansprüchen 1 bis 4 verwendet.

8. Verfahren nach Anspruch 7, dadurch gekennzeichnet, daß man die Hydroformylierung bei einer Temperatur von 30 bis 150°C und ein Druck von 1 x 10³ bis 1 x 10⁷ Pa ausführt.

9. Verfahren nach den Ansprüchen 7 und 8, dadurch gekennzeichnet, daß man als Lösungsmittel die Aldehyde verwendet, die durch die Hydroformylierung des jeweils eingesetzten Olefins entstehen.

10. Verfahren nach den Ansprüchen 7 und 8, dadurch gekennzeichnet, daß man als Lösungsmittel die hochsiedenden Verbindungen verwendet, die durch Kondensationsreaktionen der während der Hydroformylierung gebildeten Aldehyde im Zuge der Hydroformylierung entstehen, verwendet.

11. Verfahren nach den Ansprüchen 7 bis 10, dadurch gekennzeichnet, daß man α-Olefine hydroformyliert.

## Claims

1. A phosphorus-containing calixarene of the formula I 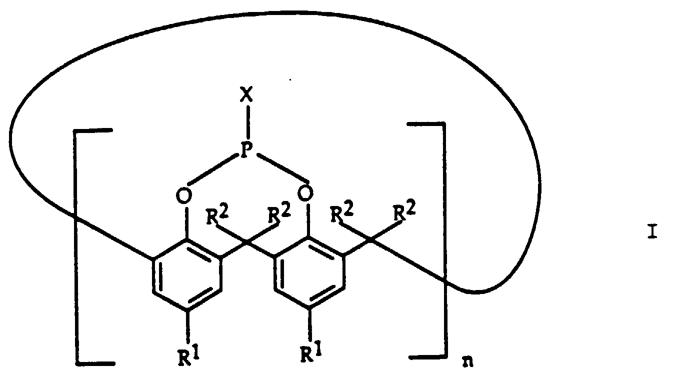 where
n is an integer from 2 to 4,
R¹ is hydrogen, C₁-C₂₀-alkyl, C₁-C₂₀-alkoxy, sulfonate or carboxylate,
the radicals R² are identical or different and are hydrogen or C₁-C₂₀-alkyl and
X is hydrogen, C₁-C₂₀-alkyl, C₁-C₂₀-alkoxy or a phenyl or phenoxy group which is unsubstituted or substituted by 1 to 3 C₁-C₂₀-alkyl, C₁-C₂₀-alkoxy, sulfonate, carboxylate, C₁-C₂₀-alkylthio or C₂-C₂₀-dialkylamino groups.

2. A phosphorus-containing calixarene as claimed in claim 1, in which X is phenyloxy of the formula II 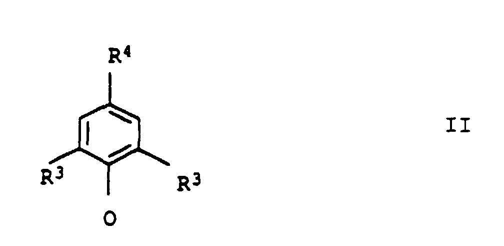 where R³ and R⁴ are identical or different and are each hydrogen, C₁-C₂₀-alkyl, C₁-C₂₀-alkoxy, C₁-C₂₀-alkylthio or C₂-C₂₀-dialkylamino and R⁴ may additionally be sulfonate or carboxylate.

3. A phosphorus-containing calixarene as claimed in claim 1, in which R¹ is hydrogen, C₁-C₆-alkyl, C₁-C₆-alkoxy, sulfonate or carboxylate, R² is hydrogen and X has the meanings stated in claim 2.

4. A phosphorus-containing calixarene as claimed in claim 1, of the formula III

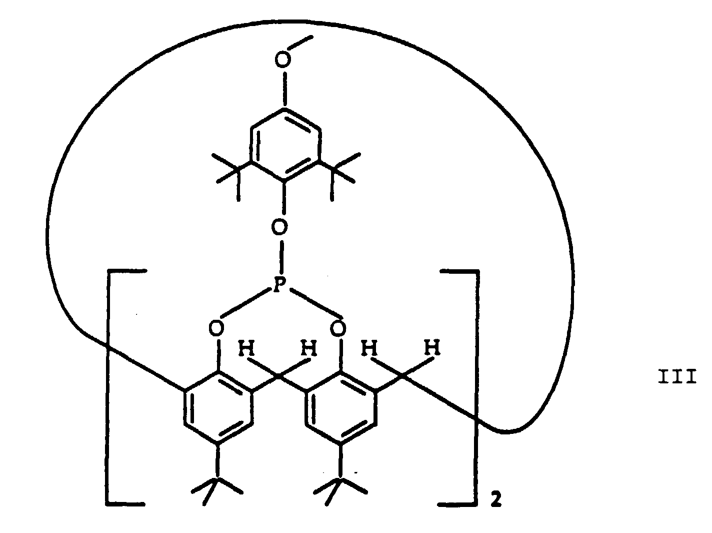

5. A process for the preparation of a phosphorus-containing calixarene as claimed in claim 1, which comprises reacting a calixarene of the formula IV 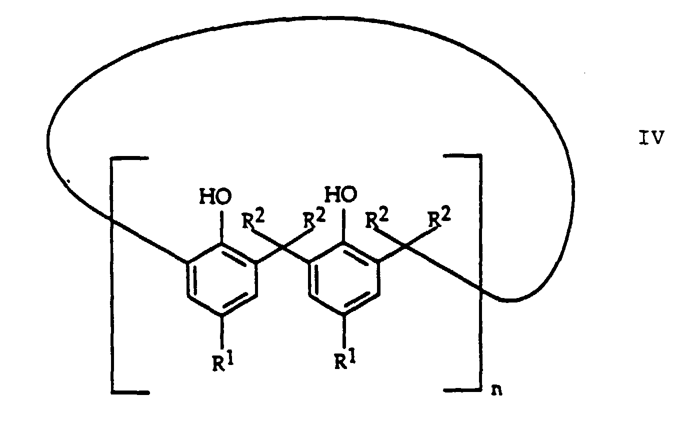 where n, R¹ and R² have the meanings stated in claim 1, with a phosphorus compound of the formula V
XPHal₂ V
where X has the meanings stated in claim 1 and Hal is fluorine, chlorine, bromine or iodine, in the presence of a base.

6. A process as claimed in claim 5, wherein the base used is a C₃-C₃₀-trialkylamine.

7. A process for the preparation of an aldehyde by the hydroformylation of a C₃-C₂₀-olefin by means of a CO/H₂ gas mixture in the presence of a rhodium or ruthenium carbonyl complex with a phosphorus-containing ligand and in the presence of a solvent, which comprises using the phosphorus-containing calixarenes as claimed in any of claims 1 to 4 as ligands.

8. A process as claimed in claim 7, wherein the hydroformylation is carried out at from 30 to 150°C and from 1 x 10³ to 1 x 10⁷ Pa.

9. A process as claimed in claims 7 and 8, wherein the solvent used is the aldehyde which is formed by the hydroformylation of the particular olefin used.

10. A process as claimed in claims 7 and 8, wherein the solvents used are the high-boiling compounds which are formed in the course of the hydroformylation by condensation reactions of the aldehydes formed during the hydroformylation.

11. A process as claimed in any of claims 7 to 10, wherein an α-olefin is hydroformylated.

## Revendications

1. Calixarènes contenant du phosphore, de la formule générale I 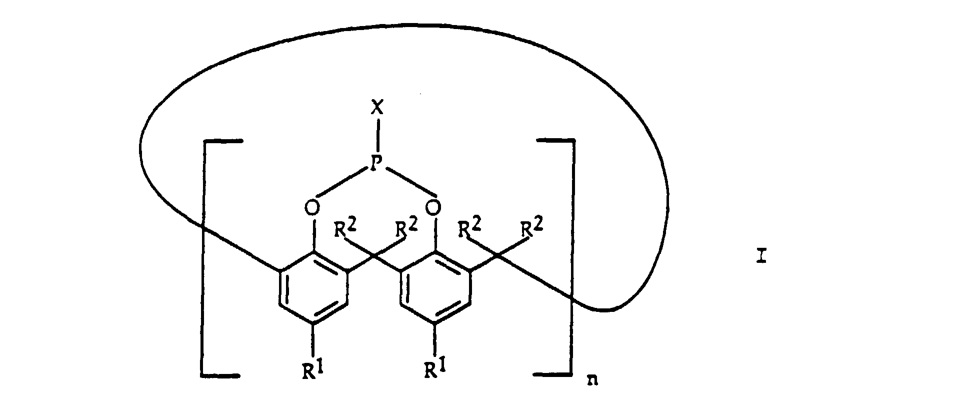 dans laquelle
n représente un nombre entier dont la valeur varie de 2 à 4,
R¹ représente un atome d'hydrogène, un radical alkyle en C₁ à C₂₀, un radical alcoxy en C₁ à C₂₀, un radical sulfonate ou un radical carboxylate, les restes
R² sont identiques ou différents et représentent chacun un atome d'hydrogène ou un radical alkyle en C₁ à C₂₀ et
X représente un atome d'hydrogène, un radical alkyle en C₁ à C₂₀, un radical alcoxy en C₁ à C₂₀, ou un groupe phénoxy ou phényle, non substitué, ou substitué par 1 à 3 radicaux alkyle en C₁ à C₂₀, radicaux alcoxy en C₁ à C₂₀, sulfonate, carboxylate, alkyl(C₁-C₂₀) thio et/ou dialkyl(C₂-C₂₀) amino.

2. Calixarènes contenant du phosphore suivant la revendication 1, dans lesquels le reste X représente un groupe phényloxy de la formule générale II 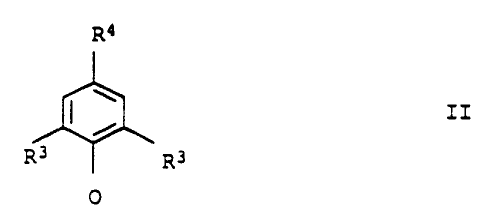 et dans laquelle le symbole R³ et le symbole R⁴ peuvent avoir des significations identiques ou différentes et représentent chacun un atome d'hydrogène, un radical alkyle en C₁ à C₂₀, un radical alcoxy en C₁ à C₂₀, un radical alkylthio en C₁ à C₂₀ et/ou un radical dialkyle(C₂-C₂₀)amino et le symbole R⁴ peut encore complémentairement représenter un radical sulfonate ou carboxylate.

3. Calixarènes contenant du phosphore suivant la revendication 1, dans lesquels le symbole R¹ représente un atome d'hydrogène, un radical alkyle en C₁ à C₆, un radical alcoxy en C₁ à C₆, un radical sulfonate, ou un radical carboxylate, le symbole R² représente un atome d'hydrogène et X possède des significations qui lui ont été attribuées dans la revendication 2.

4. Calixarène contenant du phosphore suivant la revendication 1, de la formule III.

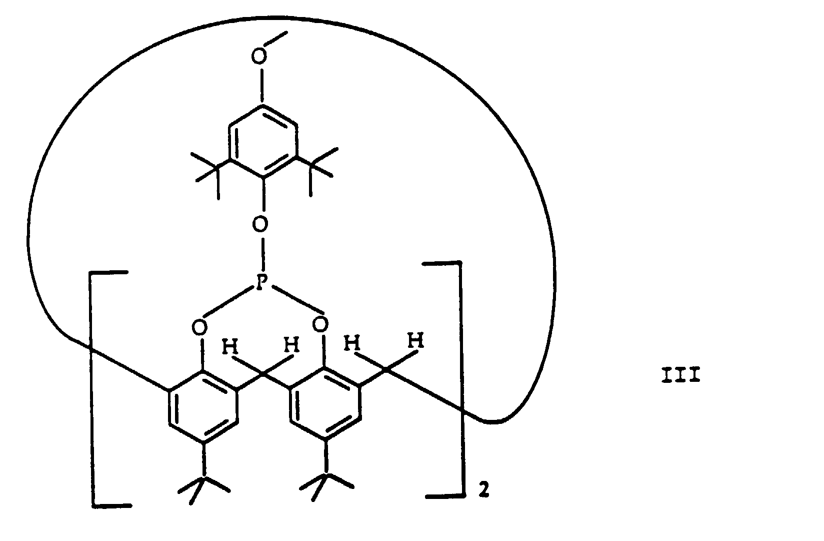

5. Procédé de préparation de calixarènes contenant du phosphore selon la revendication 1, caractérisé en ce que l'on fait réagir un calixarène de la formule générale IV 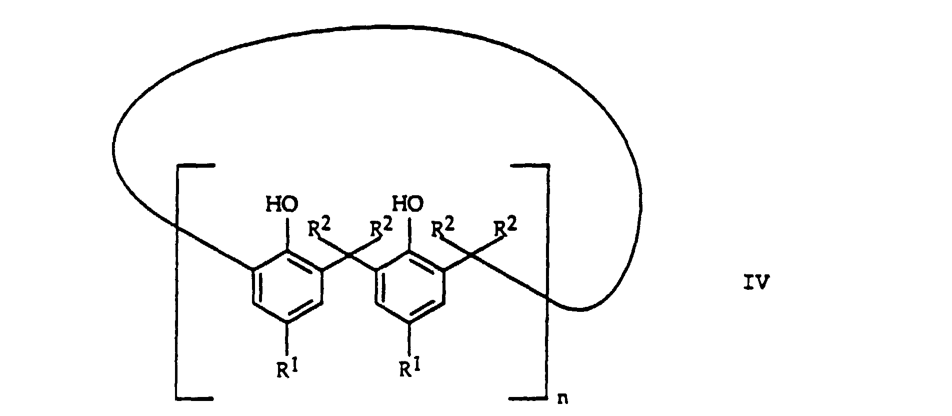 dans laquelle
n et les symboles R¹ et R² ont les significations qui leur ont été attribuées dans la revendication 1, avec un composé de phosphore de la formule générale V
XPHal₂ V
dans laquelle X possède les significations qui lui ont attribuées dans la revendication 1 et Hal représente un atome de fluor, de chlore, de brome ou d'iode, en présence d'une base.

6. Procédé suivant la revendication 5, caractérisé en ce que l'on utilise une trialkylamine en C₃ à C₃₀ à titre de base.

7. Procédé de préparation d'aldéhydes par l'hydroformylation de l'oléfine en C₃ à C₂₀ à l'aide d'un mélange gazeux CO/H₂, en présence de complexes de rhodium- ou ruthéniumcarbonyle avec un ligand contenant du phosphore et en présence d'un solvant, caractérisé en ce que l'on utilise, à titre de ligand, des calixarènes contenant du phosphore suivant l'une quelconque des revendications 1 à 4.

8. Procédé suivant la revendication 7, caractérisé en ce que l'on entreprend l'hydroformylation à une température de 30 à 150°C et sous un pression de 1 x 10³ à 1 x 10⁷ Pa.

9. Procédé suivant les revendications 7 et 8, caractérisé en ce que l'on utilise à titre de solvant, les aldéhydes qui se forment par l'hydroformylation de l'oléfine à chaque fois mise en oeuvre.

10. Procédé suivant les revendications 7 et 8, caractérisé en ce que l'on utilise à titre de solvant, des composés à points d'ébullition élevés qui se forment par des réactions de condensation des aldéhydes formés au cours de l'hydroformylation.

11. Procédé suivant les revendications 7 à 10, caractérisé en ce que l'on procède à l'hydroformylation d'α-oléfine.
